Europäisches Patentamt

European Patent Office

Office européen des brevets

(11) Veröffentlichungsnummer: **0 290 767 B1**

(12) **EUROPÄISCHE PATENTSCHRIFT**

(45) Veröffentlichungstag der Patentschrift: **31.07.91**

(51) Int. Cl.5: **A61F 2/28**, A61F 2/36, A61F 2/38

(21) Anmeldenummer: **88104938.1**

(22) Anmeldetag: **26.03.88**

(54) **Endoprothese für femorale oder tibiale Gelenkknochenteile und angrenzende Knochenabschnitte.**

(30) Priorität: **15.05.87 DE 8706999 U**

(43) Veröffentlichungstag der Anmeldung:
**17.11.88 Patentblatt 88/46**

(45) Bekanntmachung des Hinweises auf die
Patenterteilung:
**31.07.91 Patentblatt 91/31**

(84) Benannte Vertragsstaaten:
**AT CH DE FR GB IT LI NL**

(56) Entgegenhaltungen:
**EP-A- 0 144 667**
**DE-A- 3 336 004**
**DE-U- 8 706 999**
**GB-A- 2 137 884**
**US-A- 4 384 373**

(73) Patentinhaber: **Howmedica GmbH**
**Professor-Küntscher-Str. 1-5**
**W-2314 Schönkirchen ü. Kiel(DE)**

(72) Erfinder: **Kotz, Rainer, Prof.Dr.**
**Saarplatz 9/10**
**A-1190 Wien(AT)**
Erfinder: **Harder, Hans Erich**
**Mecklenburger Str. 35**
**W-2316 Probsteierhagen(DE)**

(74) Vertreter: **Dipl.-Ing. H. Hauck Dipl.-Ing. E.**
**Graalfs Dipl.-Ing. W. Wehnert Dr.-Ing. W. Dö-**
**ring**
**Neuer Wall 41**
**W-2000 Hamburg 36(DE)**

Rank Xerox (UK) Business Services

## Beschreibung

Die Erfindung bezieht sich auf eine Endoprothese für femorale oder tibiale Gelenkknochenteile und angrenzende Knochenabschnitte nach dem Oberbegriff des Patentanspruchs 1.

Aus der DE-A-33 36 004 ist eine in ihrer Länge verstellbare Endoprothese (Wachstumsprothese) bekanntgeworden, bei der eine erste mit dem Gelenkteil verbundene Hülse eine Gewindespindel drehbar, jedoch axial fest lagert, die mit einer zweiten axial verschiebbaren, ein Innengewinde aufweisenden Hülse zusammenwirkt. Diese Teleskopanordnung ist von einem Kunststoffmantel umgeben, der durch ein mit der ersten Hülse fest verbundenes hakenförmiges Element gegen Drehung gesichert ist. Ein Abschnitt der Gewindespindel ist als Schneckenrad geformt, das mit einer in der ersten Hülse drehbar gelagerten Schnecke kämmt. Die Schnecke kann von außen über ein entsprechendes Werkzeug verdreht werden, um die Endoprothese in der Länge zu verstellen.

Der Erfindung liegt die Aufgabe zugrunde, eine in der Länge verstellbare, bei einer vorgegebenen Länge feststellbare Endoprothese zu schaffen, die auch postoperativ einfach verstellt werden kann, weder während der Verstellung noch im Gebrauch eine Weichteilschädigung verursacht und zur Verstellung und Entriegelung sowie erneuter Verriegelung nur einen kleinen Eingriff erfordert.

Diese Aufgabe wird durch die Merkmale des Kennzeichnungsteils des Patentanspruchs 1 gelöst.

Bei der erfindungsgemäßen Endoprothese ist eine äußere Hülse einteilig mit dem Gelenkteil geformt, besteht mithin aus einem festen Material. Sie kann daher relativ dünn ausgeführt werden, so daß der Absatz zwischen der äußeren und der Spindelmutterhülse relativ gering ist und eine Verstellung der Spindelmutterhülse gegenüber der äußeren Hülse keine Beeinträchtigung der Weichteile mit sich bringt.

Bei der erfindungsgemäßen Endoprothese sind die Spindelmutter und der Antriebszapfen für das Winkelgetriebe im Gelenkteil gelagert. Da im Bereich des Gelenkteils nur eine geringe Weichteildeckung besteht, ergibt sich ein sehr einfacher Zugang zum Antriebszapfen.

Bei der erfindungsgemäßen Endoprothese ist eine Verriegelung des Winkelgetriebes vorgesehen, so daß eine einmal eingestellte Länge unverrückbar beibehalten wird. Zu diesem Zweck wirkt ein Feststellstift, der im Gelenkteil verschraubt ist, mit einem Bund des Kegelrades zusammen, das drehfest auf der Gewindespindel sitzt. Feststellstift und Antriebszapfen können daher unmittelbar benachbart sein, so daß der Gesamteingriff, der für eine Längenverstellung erforderlich ist, minimal ist.

Eine Drehsicherung der Spindelmutterhülse gegenüber der äußeren Hülse läßt sich erfindungsgemäß auf einfache Weise dadurch erreichen, daß die äußere Hülse einen nach innen weisenden Vorsprung aufweist, der mit einer Nut der Spindelmutterhülse zusammenwirkt. Durch die erfindungsgemäße Drehsicherung wird die Dicke der Endoprothese in dem Bereich nicht vergrößert.

Die erfindungsgemäße Endoprothese besteht aus einer Mindestanzahl von Bauteilen, die einfach zu fertigen und zu montieren sind.

Vorteilhafte Ausgestaltungen der Erfindung sind in den Unteransprüchen angegeben.

Ausführungsbeispiele der Erfindung werden nachfolgend anhand von Zeichnungen näher erläutert.

Fig. 1     zeigt eine Seitenansicht teils im Schnitt einer ersten Ausführungsform der Endoprothese nach der Erfindung.

Fig. 2     zeigt eine Seitenansicht des unteren Teils der Endoprothese nach Fig. 1 in Richtung Pfeil 2.

Fig. 3     zeigt vergrößert einen Schnitt durch den tibialen Abschnitt der Endoprothese nach Fig. 1, jedoch in einer abgewandelten Ausführungsform.

Fig. 4     zeigt eine Seitenansicht teilweise im Schnitt eines Gelenkteils zur Verbindung mit der Endoprothese nach Fig. 1.

Fig. 5     zeigt eine Seitenansicht des unteren Teils der äußeren Hülse der Endoprothese nach Fig. 1.

Fig. 6     zeigt einen Schnitt durch Fig. 5 entlang der Linie 6-6.

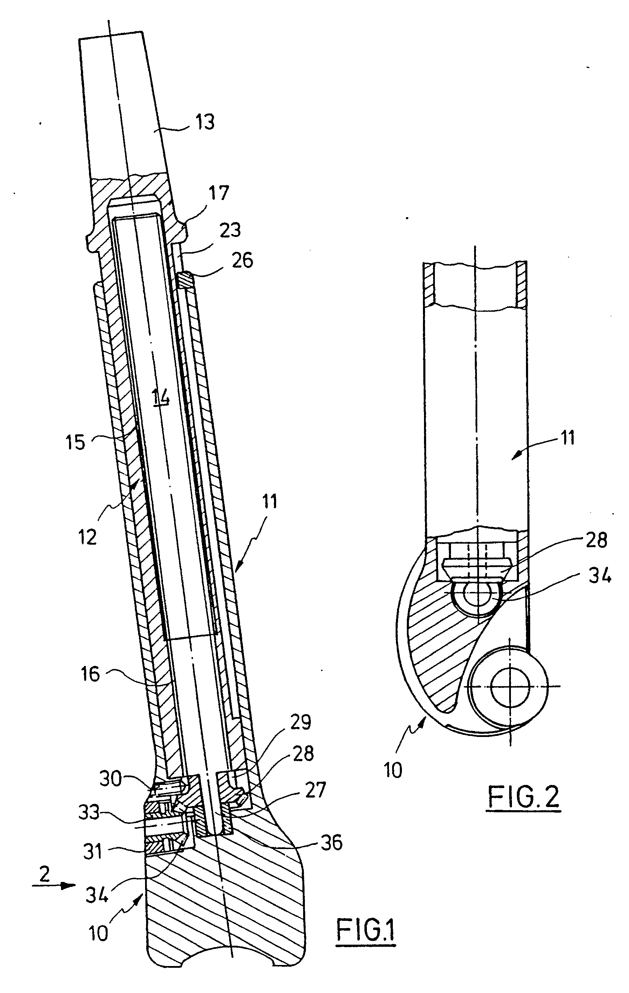

Die in Fig. 1 dargestellte Endoprothese besitzt ein femorales Kniegelenkteil 10, auf dessen Aufbau im einzelnen nicht eingegangen werden soll. Es wirkt zusammen mit einem nicht gezeigten tibialen Gelenkteil. Mit dem Gelenkteil 10 ist eine äußere Hülse 11 verbunden. Teleskopisch innerhalb der Hülse verschiebbar ist eine Spindelmutterhülse 12, die am äußeren Ende einen Außenkonus 13 aufweist. Innerhalb der Hülse 12 befindet sich eine Gewindespindel 14.

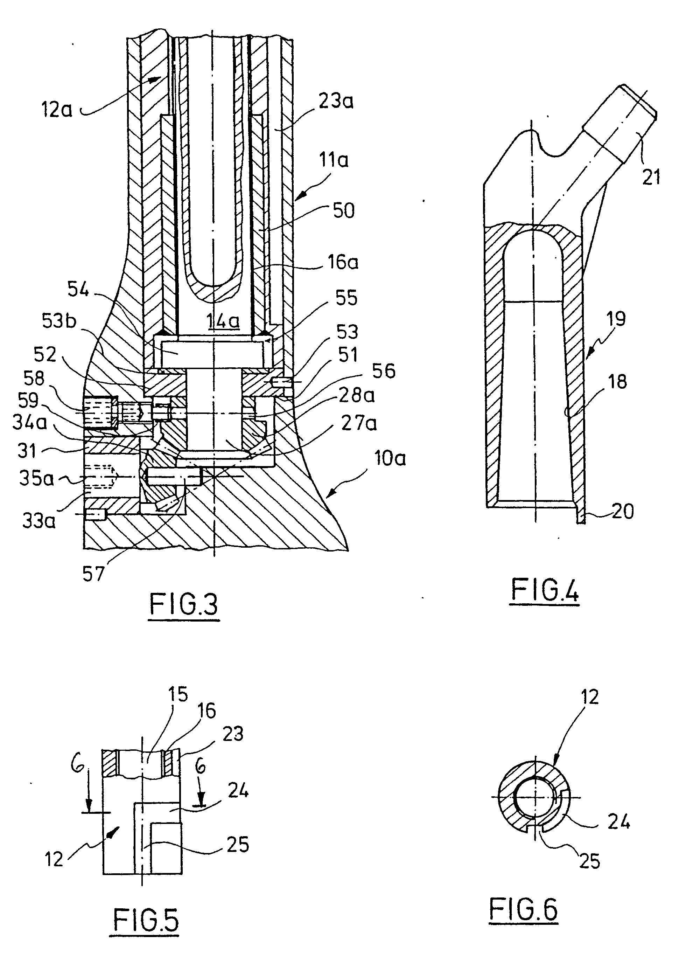

Der Aufbau der Mutterhülse 12 geht aus den Figuren 3, 4 und 6 näher hervor. Die Innenbohrung 15 der Hülse 12 weist nur nahe dem offenen Ende einen Gewindeabschnitt 16 auf. Der Durchmesser der Bohrung 15 ist etwas größer als der Innendurchmesser des Gewindeabschnitts 16. Unterhalb des Konus 13 ist ein Bund 17 geformt. Der Konus 13 dient zum Beispiel zur Aufnahme des Innenkonus 18 eines femoralen Hüftgelenkteils 19 gemäß Fig. 4. Die Konen 13, 18 wirken selbsthemmend zusammen. Eine Nase 20 des Gelenkteils 19 wirkt mit einer entsprechenden Ausnehmung am Konus 13 zusammen und legt somit die Drehlage der beiden Teile zueinander fest. Das Gelenkteil 19 weist einen weiteren Konus 21 auf zur Aufnahme einer Gelenkkugel. Anstelle des Gelenkteils 19 kann auch ein Anschlußstück bzw. ein Verlängerungsstück treten. Im ersteren Fall würde der Konus 13 durch einen Innenkonus ersetzt, der mit einem Außenkonus eines nicht gezeigten Anschlußstücks zusammenwirkt.

Die Hülse hat an der Außenseite eine achsparallele Längsnut 23. Sie endet in einem in Umfangsrichtung sich erstreckenden Nutabschnitt 24, der am anderen Ende in einen achsparallelen Längsnutabschnitt 25 mündet. Wie aus Fig. 1 ersichtlich, besitzt die äußere Hülse 11 am freien Ende einen radial nach innen gerichteten Vorsprung 26, der in die Längsnut 23 eingreift. Beim Einführen der Mutterhülse 12 in die äußere Hülse 11 gelangt der Vorsprung 26 zunächst in den unteren Nutabschnitt 25. Anschließend wird die Hülse 12 um einen gewissen Betrag gedreht (Vierteldrehung) bis der Vorsprung in die Nut 23 eintreten kann.

Die Gewindespindel 14 ist am unteren Ende mit einem Zapfen 27 versehen, auf den drehfest ein Kegelrad 28 aufgeschoben ist. Der Zapfen 27 ragt jedoch über das Kegelrad 28 hinaus. Im Kegelrad 28 sind einzelne in Umfangsrichtung versetzte Vertiefungen 29 vorgesehen. In Fig. 1 ist ein in das Gelenkteil 10 eingesetzter Gewindestift 30 zu erkennen, der in eine Nut ragt, die zwischen dem Kegelrad 28 und dem unteren Ende der Hülse 12 gebildet ist. Im gezeigten Fall greift dieser Gewindestift 30 in eine Vertiefung 29 ein und verhindert mithin eine Verdrehung der Gewindespindel 14.

In eine Bohrung des Gelenkteils 10 ist eine Lagerbuchse 31 eingeschraubt. Sie dient zur Lagerung eines Zapfens 33, der am inneren Ende ein Kegelrad 34 aufweist, das mit dem Kegelrad 28 der Gewindespindel 14 kämmt. Der Zapfen weist einen Innensechskant auf, in den ein entsprechendes Drehwerkzeug eingreifen kann.

Der über das Kegelrad 28 überstehende Teil des Zapfens 27 greift in eine Lagerbuchse 36 ein, die im Gelenkteil 10 angeordnet ist. Das Kegelrad 28 gleitet mithin auf der zugekehrten Stirnseite der Lagerbuchse 36.

Man erkennt aus Fig. 1, daß eine Drehung des Antriebszapfens 33 zu einer Drehung des Kegelrads 34 führt, das seinerseits das Kegelrad 28 und damit die Gewindespindel 14 in Drehung versetzt. Dadurch wird die Mutterhülse 12 axial bewegt und gleitet beispielsweise aus der äußeren Hülse 11 heraus. Man erkennt ferner, daß die Lage des Innengetriebes derart ist, daß der Antriebszapfen 33 sehr leicht erreichbar ist. Im Bereich des Knies liegt bekanntlich eine geringe Weichteildeckung vor.

Der Antriebsmechanismus gemäß Fig. 3 unterscheidet sich in einigen Punkten von dem nach Fig. 1. Soweit mit Fig. 1 gleiche Teile verwendet werden, sind diese in Fig. 3 mit gleichen Bezugszeichen versehen, die jedoch zusätzlich den Index a tragen.

In einem erweiterten Abschnitt der Gewindehülse 12a ist eine Hülse 50 eingeschoben und durch Schweißung befestigt. Sie ist mit dem Gewindeabschnitt 16a versehen, der mit dem Gewinde der Gewindespindel 14a zusammenwirkt. Aus Fig. 3 ist auch zu erkennen, daß die Gewindespindel aus Gewichts- und Ersparnisgründen hohl ist.

Die Bohrung des Schaftes 11 weist nahe dem Gelenkteil 10a eine Schulter 51 auf, auf der ein Ring 52 ruht, der mit Hilfe eines Stifts 53, der durch die Wandung des Schaftes 11a hindurchgeführt ist, festgelegt ist. In die obere Stirnseite des Ringes 52 ist eine Gleitscheibe 53 bündig eingelassen. Der zylindrische Zapfen 27a der Gewindespindel 14a erstreckt sich durch die Bohrung des Ringes 52. Oberhalb des Zapfens 27a ist ein Ringbund 54 an die Gewindespindel angeformt. Der Ringbund befindet sich innerhalb eines freien Raums 55, der durch eine Durchmessererweiterung der Bohrung der Hülse 12a gebildet ist unterhalb der Hülse 50. Die Gewindespindel 14a stützt sich mithin über den Ringbund 54 auf dem Ring 52 ab, der mithin die axiale Belastung auf das Gelenkteil 10a überträgt.

Das Zahnrad 28a ist mit Hilfe von Stiften 56 am Zapfen 27a festgelegt. Es ist frei von axialen Belastungen. Es kämmt mit dem Zahnrad 34a, das einteilig mit dem Zapfen 33a geformt ist. Der Zapfen 33a ist in der Gewindehülse 31a drehbar gelagert und weist eine Sechskantausnehmung 35a für ein Drehwerkzeug auf. Die Rückseite des im Durchmesser größeren Zahnrads 34a sichert das Zahnrad 34a bzw. den Zapfen 33a gegen eine axiale Bewegung nach außen. Gegenüber dem Zapfen 33a ist ein Lagerstift 57 mittig in das Zahnrad 34a eingelassen zur verbesserten axialen Lagerung des Zahnrads 34a.

Im Gelenkteil 10a ist ein im Außendurchmesser gestufter Feststellstift oder eine Feststellschraube 58 von einer entsprechenden Gewindebohrung aufgenommen. Das innere Ende hält ein im Querschnitt U-förmiges Ringsegment zur Festsetzung des Zahnrads 28a. Das Ringsegment ermöglicht eine Verteilung der Druckkraft über einen gewissen Umfangsbereich des Zahnrads 28a.

**Patentansprüche**

1. Endoprothese für femorale oder tibiale Gelenkknochenteile und angrenzende femorale oder tibiale Knochenabschnitte, mit einem femoralen Hüftgelenkteil und/oder einem femoralen Kniegelenkteil und/oder einem tibialen Kniegelenkteil und einem den Knochenabschnitt ersetzenden Knochenersatzteil, das von einer längeren Spindelmutterhülse (12) mit Innengewinde und einer äußeren mit dem Gelenkteil (10) verbundenen Hülse (11) gebildet ist, die teleskopisch zusammenwirken, wobei zur Längenverstellung des Knochenersatzteils innerhalb der Spindelmutterhülse (12) eine axial fest, jedoch drehbar gelagerte Gewindespindel (14) angeordnet und über ein Winkelgetriebe (28, 34) mit einem Antriebszapfen (33, 33a) verbunden ist, dadurch gekennzeichnet, daß das Winkelgetriebe zwei kämmende Kegelräder (28, 34; 28a, 34a) aufweist, von denen eines (28, 28a) drehfest mit der Gewindespindel (14, 14a) drehfest verbunden ist und das andere (34, 34a) drehbar im Gelenkteil (10, 10a) gelagert ist, die äußere Hülse (11, 11a) einteilig mit dem Gelenkteil (10, 10a) geformt ist, die Gewindespindel (14, 14a) und der Antriebszapfen (33, 33a) drehbar im Gelenkteil (10, 10a) gelagert sind, die äußere Hülse (11, 11a) einen nach innen weisenden, vorzugsweise am Ende angebrachten Vorsprung (26) aufweist, der in eine Längsnut (23) der Spindelmutterhülse (12, 12a) eingreift und neben dem Antriebszapfen (33, 33a) ein Feststellstift (30, 58) in das Gelenkteil (10, 10a) geschraubt ist, der mit einem Bund des einen Kegelrads (28, 28a) zusammenwirkt.

2. Endoprothese nach Anspruch 1, dadurch gekennzeichnet, daß die Nut (23) an ihrem inneren Ende einen in Umfangsrichtung versetzten Endabschnitt (25) aufweist, der durch einen in Umfangsrichtung verlaufenden Nutabschnitt (24) mit der übrigen Längsnut (23) verbunden ist.

3. Endoprothese nach einem der Ansprüche 1 oder 2, dadurch gekennzeichnet, daß die Gewindespindel (14) an dem inneren Ende einen Lagerzapfen (27) aufweist, der von einer Lagerbuchse (36) im Gelenkteil (10) aufgenommen ist.

4. Endoprothese nach Anspruch 3, dadurch gekennzeichnet, daß ein Kegelrad (28) auf dem Zapfen (27) aufgenommen ist, dessen Stirnseite mit der Stirnseite der Lagerbuchse (36) zusammenwirkt.

5. Endoprothese nach einem der Ansprüche 1 bis 4, dadurch gekennzeichnet, daß der mit dem Kegelrad (34) versehene Antriebszapfen (35) in einer Lagerbuchse (31) gelagert ist, die eine Begrenzung (32) des Antriebszapfens (33) aus der Lagerbuchse heraus vorsieht.

6. Endoprothese nach Anspruch 5, dadurch gekennzeichnet, daß die Lagerbuchse (31) in eine Gewindebohrung des Gelenkteils (10) eingeschraubt ist.

7. Endoprothese nach einem der Ansprüche 3 bis 6, dadurch gekennzeichnet, daß zwischen dem Kegelrad (28) auf der Spindel (14) und einer radialen Schulter der Spindel (14) eine umlaufende Nut gebildet ist, die mindestens eine Vertiefung (29) aufweist und der Gewindestift (30) in die Nut eingreift.

8. Endoprothese nach einem der Ansprüche 1 bis 7, dadurch gekennzeichnet, daß nur ein begrenzter, dem Winkelgetriebe benachbarter Abschnitt (16) der Mutterhülse (12) mit einem Innengewinde versehen ist.

9. Endoprothese nach einem der Ansprüche 1 oder 2, dadurch gekennzeichnet, daß die Gewindespindel (14a) einen Ringbund (54) aufweist, der mit einem Lagerring (52, 53) im Inneren des Gelenkteils (10a) zusammenwirkt.

10. Endoprothese nach Anspruch 1, dadurch gekennzeichnet, daß der Feststellstift (58) am inneren Ende mit einem Ringsegment (59) versehen ist, das sich gegen den zylindrischen Bund des Zahnrads (28a) legt.

## Claims

1. Endoprosthesis for femoral or tibial bone joint components and adjacent femoral or tibial bone. parts, including a femoral hip joint component and/or a femoral knee joint component and/or a tibial knee joint component and a replacement part replacing said bone part, which replacement part is defined by an elongated spindle nut sleeve (12) having an internal thread and an outer sleeve (11) connected to said joint component (10), both said sleeves telescopically cooperating, wherein an axially secured, but rotatably supported threaded spindle (14) is provided within the spindle nut sleeve for longitudinally adjusting said replacement part, which threaded spindle is connected through an angular gear (28,34) to a drive pin (33,33a), characterized in that said angular gear comprises a pair of meshing bevel wheels (28,34;28a,34a), one (28,28a) being positively secured to said threaded spindle (14,14a) and the other (34,34a) being rotatably supported in said joint component (10,10a) that said outer sleeve (11,11a) and said joint component (10,10a) are integrally formed, that said threaded spindle (14,14a) and said drive pin (33,33a) are rotatably supported in said joint component (10,10a), that the outer sleeve (11,11a) preferably at the end thereof includes a projection (26) inwardly facing and engaging a longitudinal groove (23) of the spindle nut sleeve (12,12a), and that a fixing pin (30,58) is screwed in said joint component (10,10a) adjacent said drive pin (33,33a) which fixing pin cooperates which a flange of the one bevel wheel (28,28a).

2. The endoprosthesis of claim 1, characterized in that an inner end of said groove (23) comprises a peripherally offset end portion (25) which comunicates with said longitudinal groove (23) via a peripheral groove portion (24).

3. The endoprosthesis of one of claims 1 or 2, characterized in that an inner end of said threaded spindle (14) comprises a supporting pin (27) which is received by a bearing bush (36) in said joint component (10.)

4. The endoprosthesis of claim 3, characterized in that a bevel wheel (28) is received on a pin (27) with a front face thereof cooperating with a front face of said bearing bush (36).

5. The endoprosthesis of one of claims 1 to 4, characterized in that said drive pin (35) including said bevel wheel (34) is supported in a bearing bush (31) providing a limitation (32) of said drive pin (33) out of said bearing bush.

6. The endoprosthesis of claim 5, characterized in that said bearing bush (31) is screwed in a threaded bore of said joint component (10).

7. The endoprosthesis of one of claims 3 to 6, characterized in that a rotary groove is formed between said bevel wheel (28) on said spindle (14) and a radial shoulder of said spindle (14) which groove includes at least a depression (29) and that said threaded pin (30) engages said groove.

8. The endoprosthesis of one of claims 1 to 7, characterized in that a limited portion (16) of said nut sleeve (12) adjacent said bevel gear is provided with an internal thread.

9. The endoprosthesis of one of claims 1 or 2, characterized in that said threaded spindle (14a) includes an annular flange (54) cooperating with a bearing ring (52,53) within said joint component (10a).

10. The endoprosthesis of claim 1, characterized in that an inner end of said fixing pin (58) is provided with an annular segment (59) contacting said cylindrical flange of said bevel wheel (28a).

**Revendications**

1. Endoprothèse pour parties d'articulation fémorales ou tibiales et pour des segments osseux fémoraux ou tibiaux voisins, comprenant une partie d'articulation fémorale de la hanche et/ou une partie d'articulation fémorale du genou et/ou une partie d'articulation tibiale du genou, et une pièce de remplacement d'os, remplaçant le segment osseux, qui est constituée par un manchon-écrou assez long (12) de broche, pourvu d'un filetage intérieur, et par un manchon extérieur (11) relié à la partie d'articulation, (10), qui coopèrent de manière télescopique,dans laquelle, pour régler la longueur de la pièce de remplacement d'os, une broche filetée (14) est montée en rotation, mais axialement fixe, à l'intérieur du manchon-écrou de broche (12) et est reliée à un tenon d'entraînement (33, 33a) au moyen d'un engrenage angulaire (28, 34), caractérisée en ce que :
   - l'engrenage angulaire présente deux pignons coniques (28, 34; 28a, 34a) s'engrenant l'un dans l'autre, dont l'un (28 ; 28a) est fixé à la broche filetée (14 ; 14a) sans rotation possible et l'autre (34 ; 34a) est monté à rotation dans la partie d'articulation (10 ; 10a)
   - le manchon extérieur (11 ; 11a) est réalisé d'une seule pièce avec la partie d'articulation (10 ; 10a),
   - la broche filetée (14 ; 14a) et le tenon d'entraînement (33 ; 33a) sont montés à rotation dans la partie d'articulation (10 ; 10a),
   - le manchon extérieur (11 ; 11a) présente une partie saillante (26), disposée de préférence à son extrémité et tournée vers l'intérieur, qui s'engage dans une rainure longitudinale (23) du manchon-écrou de broche (12 ; 12a) et,
   - à côté du tenon d'entraînement (33 ; 33a), une vis de blocage (30 ; 58) est vissée dans la partie d'articulation (10 ; 10a) et coopère avec un épaulement d'un pignon conique (28 ; 28a).

2. Endoprothèse selon la revendication 1, caractérisée en ce que la rainure (23) présente à son extrémité intérieure une section terminale .(25) décalée dans la direction périphérique, qui est reliée au reste de la rainure longitudinale (23) par une section de rainure (24) orientée dans la direction périphérique.

3. Endoprothèse selon la revendication 1 ou 2, caractérisée en ce que la broche filetée (14) présente à son extrémité intérieure un tenon (27) qui est reçu par un coussinet (36) de la partie d'articulation (10).

4. Endoprothèse selon la revendication 3, caractérisée en ce qu'un pignon conique (28) est monté sur le tenon (27), sa face frontale coopèrant avec le côté frontal du coussinet (36).

5. Endoprothèse selon l'une des revendications 1 à 4, caractérisée en ce que le tenon d'entraînement (33) pourvu du pignon conique (34) est logé dans un coussinet (31) qui s'oppose à un déplacement du tenon d'entraînement (33) vers l'extérieur.

6. Endoprothèse selon la revendication 5, caractérisée en ce que le coussinet (31) est vissé dans un taraudage de la partie d'articulation (10).

7. Endoprothèse selon l'une des revendications 3 à 6, caractérisée en ce qu'une rainure périphérique est formée entre le pignon conique (28) situé sur la broche (14) et un épaulement radial de la broche (14), ladite rainure présentant au moins un renfoncement (29), et la vis (30) s'engage dans la rainure

8. Endoprothèse selon l'une des revendications 1 à 7, caractérisée en ce qu'une section limitée (16) du manchon-écrou (12), voisine de l'engrenage angulaire, comporte un filet intérieur.

9. Endoprothèse selon l'une des revendications 1 ou 2, caractérisée en ce que la broche filetée (14a) présente un épaulement annulaire (54) qui coopère avec une bague de coussinet (52, 53) à l'intérieur de la partie d'articulation (10a).

10. Endoprothèse selon la revendication 1, caractérisée en ce que la goupille de blocage (58) est pourvue à son extrémité intérieure d'un segment annulaire (59) qui s'appuie contre l'épaulement cylindrique du pignon (28a).

13

17

23

26

14

15

12

16

11

29

28

30

27

33

36

31

2

34

10

FIG.1

11

28

34

10

FIG.2

7

12a 23a 11a 50 16a 54 55 53b 53 52 51 58 56 59 28a 34a 27a 31 10a 35a 33a 14a 57

FIG.3

21 19 18 20

FIG.4

15 16 23 24 25 6 6 12

FIG.5

12 24 25

FIG.6